# FASCICULE DE BREVET EUROPEEN

(11) **EP 0 533 739 B1**
(45) Date de publication et mention de la délivrance du brevet: **10.08.1994**
(21) Numéro de dépôt: 91910762.3
(22) Date de dépôt: 12.06.1991
(51) Int. Cl.: A61K 9/16

(54) **COMPOSITION PHARMACEUTIQUE INJECTABLE**
INJIZIERBARES ARZNEIMITTEL
INJECTABLE PHARMACEUTICAL COMPOSITION

(30) Priorité: 14.06.1990 FR 9007416
(43) Date de publication de la demande: 31.03.1993
(73) Titulaire: APLICACIONES FARMACEUTICAS, S.A. DE C.V., Mexico 03100 (MX)
(72) Inventeur: JOSUE, Garza, Flores, 54050 Mexico (MX); LAISECA SOTO, Laura, P., Rodeo 08110 Mexico (MX); GUILLEN PICHARDO, Jose, Guerrero 06000 Mexico (MX); ANGELES URIBE, Juan, Valle de Aragon 57100 Mexico (MX)
(74) Mandataire: Kiliaridis, Constantin
(86) Numéro de dépôt international: EP9101096
(87) Numéro de publication internationale: WO9119484

(56) Documents cités:
- EP-A- 0 257 368
- WO-A-90/13285
- BE-A- 670 438
- FR-A- 2 070 153

## Description

La présente invention concerne un procédé pour améliorer le contrôle des propriétés pharmacocinétiques et pharmacologiques de substances pharmaceutiquement actives. Elle concerne également des particules de substances actives, et leur utilisation dans des formulations injectables à libération retardée.

### Art antérieur

Des substances biologiquement actives, faiblement solubles en milieu physiologique, ont déjà été utilisées sous forme de suspension de particules et administrées par injection intramusculaire pour obtenir une dissolution lente, donc un effet prolongé dans l'organisme humain ou animal. Par exemple, des mélanges de norethistérone et de mestranol, sous forme de poudre cristalline en suspension acqueuse, ont été essayés pour la fabrication d'un anticonceptif injectable intramusculaire(J. Garza Flores et al, Contraception, mai 1988, Vol. 35, N° 5, 471 - 481).

Probablement à cause des variations de granulométrie et des irrégularités de forme des particules, ces compositions de l'art antérieur présentent en général plusieurs défauts :
- Courbe de libération des substances actives présentant un fort pic juste après l'injection, puis une pente descendante, ce qui augmente la dose totale nécessaire pour obtenir un effet durable suffisant.
- Formation occasionnelle de grumeaux ou croûtes dans la suspension.
- Nécessité d'utiliser des aiguilles hypodermiques de grand diamètre pour éviter le risque d'un blocage en sortie de seringue.

Le brevet FR 2 070 153 (DUPONT DE NEMOURS) décrit des suspensions de particules de principes actifs enrobées de matrices de polymères polylactides. Cette technique diminue l'effet de choc médicamenteux initial et ralentit la libération de la substance active. Cependant, les irrégularités de forme créent, là aussi, un risque d'incident opératoire au moment de l'injection, et les variations de forme, de taille et de composition interne de ces particules entraînent une variabilité non souhaitable des vitesses de dissolution dans l'organisme receveur, c'est-à-dire une dispersion des résultats ne permettant pas une prédiction pharmacocinétique précise.

Le brevet EP N° 257 368 (AMERICAN CYANAMID CO) décrit une composition à usage parentéral constituée de microsphères de graisses et/ou de cires, d'origines naturelle ou synthétique, à bas point de fusion (40-60°C), chargées de particules d'un polypeptide, par exemple une hormone de croissance. Lorsque ces compositions sont injectées à des bovins, l'hormone de croissance voit sa dissolution retardée par l'enrobage de cire ou de graisse, ce qui entraîne une prolongation de sa présence dans l'organisme animal, entraînant une augmentation de la croissance ou de la lactation. Ces microsphères ont tendance à se déformer, s'agglutiner ou à coalescer lorsque la température ambiante est élevée, notamment dans les pays tropicaux (40-60°C), ce qui peut entraîner des problèmes de manutention ou de stockage. Comme la proportion de polypeptide actif, dans la particule, est limitée en pratique à 30 - 40 %, l'injection de ces particules présente également l'inconvénient d'introduire dans l'organisme une quantité de substance-porteur étrangère et inutile à cet organisme, au moins de l'ordre de 1,5 - 3 fois celle de la substance active.

D'autres techniques d'enrobage ou de micro-encap-sulations ont été utilisées dans l'art antérieur dont une partie est décrite, par exemple dans "Encyclopédia of Chemical Technology, 3e édition, volume 15, pages 470 à 493 (1981), JOHN WILEY AND SONS. Les micro-capsules ainsi formées contiennent souvent des particules "centrales" de taille très différente, voire pas de particule centrale du tout. Les micro-sphères ou micro-capsules de l'art antérieur permettent une dissolution ralentie, donc une libération globalement retardée des principes actifs. Cependant, compte tenu des hétérogénéités de forme et de masse des particules centrales ou des particules ultra fines dispersées pouvant se trouver enrobées dans des capsules de dimension extérieure semblable, la vitesse de libération du principe actif n'est pas homogène et un contrôle fin de la libération, voire une libération finement programmée en fonction du temps n'est pas possible.

D'autre part, sur le plan pharmacologique, la reproductibilité et la fiabilité des résultats obtenus avec ces préparations de l'art antérieur n'est pas suffisante, pour certaines applications comme par exemple la contraception, ce qui constitue un obstacle à leur emploi, en pratique, sur une grande échelle.

Une telle libération programmée est cependant souhaitable, en particulier lorsque l'action de la substance biologiquement active doit coïncider avec un cycle biologique naturel de l'organisme humain ou animal (par exemple menstruel) ou lorsqu'il est important (par exemple dans le cas d'un analgésique, d'un alcaloïde, d'un tonicardiaque, etc.) que les taux de libération soient bien contrôlés, pour éviter toute période de surdose ou au contraire de sous-dosage au moment d'une injection suivant une injection antérieure.

### Sommaire de l'invention

Le but de la présente invention est de fournir des formulations à libération retardée pour l'administration par injection parentérale, destinées par exemple aux applications citées dans le paragraphe précédent, qui permettent un contrôle fin de cette libération sans présenter les inconvénients des suspensions de particules ou des microcapsules de l'art antérieur.

Ce but est atteint grâce à l'emploi de microsphères solides, non poreuses et calibrées, constituées substantiellement de substances pharmaceutiquement actives.

La vitesse de dissolution d'une microsphère dans un milieu-solvant donné (milieu visé de préférence : le milieu physiologique intérieur) est essentiellement fonction du rayon de la sphère, compte tenu des relations liant volume, surface et rayon d'une sphère.

Selon un aspect de la présente invention, le fait d'utiliser des sphères solides, non poreuses permet d'avoir une connaissance précise de la relation masse-surface des particules et donc, grâce à une sélection du calibre des sphères, c'est-à-dire du rayon ou d'une répartition de rayons, d'avoir un contrôle précis du taux de libération du ou des principes actifs administrés. Cette même précision de contrôle, en évitant les surdosifications ou le besoin de compenser des sous-dosifications, permet de réduire l'administration totale de la ou des substances biologiquement actives à la quantité minimum requise pour obtenir l'effet thérapeutique désiré et de diminuer ainsi le risque de produire chez le malade des effets secondaires indésirables.

Utilisées sous forme de principes actifs purs, les microsphères selon la présente invention présentent l'avantage par rapport aux particules enrobées ou micro-encapsulées de l'art antérieur de diminuer le volume de matière solide devant être injectée à un organisme vivant. Elles présentent également l'avantage de ne pas introduire d'excipient solide inutile plus ou moins dégradable dans l'organisme.

Elles présentent également l'avantage de ne pas utiliser d'exipient à bas point de fusion (< à 60°C) susceptible de faire s'aglutiner les sphères entre elles et de provoquer des incidents au moment de l'injection.

Certaines substances peuvent être associées à des adjuvants non directement actifs sur l'organisme receveur : l'association peut comprendre divers moyens additifs pharmaceutiquement acceptables pour améliorer la stabilité ou l'intégrité chimique des substances biologiquement actives, étant entendu qu'il ne s'agit pas d'excipients de type vecteur. En particulier, il peut s'avérer utile d'abaisser le point de fusion ou d'inhiber une réaction de décomposition durant le procédé de fabrication (par exemple le procédé par fusion-congélation) des microsphères.

Par rapport aux suspensions de principes actifs purs sous forme de particules de formes irrégulières connues dans l'art antérieur, les microsphères selon la présente invention ont l'avantage d'avoir moins tendance à s'agglutiner et de passer de manière plus fluide par une aiguille hypodermique. D'autre part, des microsphères peuvent être triées et séparées de manière plus fine et plus fiable en fonction de leur taille, que des particules de formes irrégulières.

La formulation selon la présente invention peut se présenter sous forme de poudre de microsphères en flacons-ampoules, prête à être mise en suspension, ou sous forme de suspension déjà préparée en ampoules injectables prêtes à être administrées en médecine humaine ou vétérinaire. Le milieu de suspension peut être de l'eau, une solution saline, une huile, contenant les tampons, surfactants, conservants, habituellement utilisés dans les suspensions injectables par les pharmaco-techniciens, ou tout autre substance ou combinaison qui ne menace pas l'intégrité physique et chimique des substances en suspension et qui convienne pour l'organisme qui la recevra. Si l'on souhaite éviter une élévation initiale brusque du taux de principe actif dans le milieu intérieur de l'organisme receveur, on préfèrera, dans le cas des suspensions prêtes à l'emploi, l'utilisation de vecteurs liquides dans lequel lesdits principes actifs sont pratiquement insolubles. Dans le cas de substances actives partiellement solubles dans le vecteur liquide tiède, mais insolubles à froid, on préfèrera, sur le plan pharmacologique, éviter la formation de précipités (dit effet de "caking") en réalisant des formulations présentant séparément les microsphères en poudre et le vecteur liquide qui ne seront mélangés qu'au moment de l'injection.

Dans les applications vétérinaires, où la durée d'effet désirée peut être très longue (par exemple période de lactation de la femelle adulte), on peut utiliser des diamètres de quelques centaines de micromètres (microns). Si on souhaite limiter le diamètre des aiguilles de seringues d'injection pour le confort du patient, il est bon de limiter le diamètre des microsphères à 300 µm et plus préférablement à 100 µm. Par contre, pour des durées d'effet désiré très courtes (par exemple circadiennes), le diamètre de la microsphère peut s'abaisser à 1 µm.

Pour la plupart des applications en médecine humaine (durée d'action du principe actif comprise entre un cycle circadien et un cycle mensuel), il est préférable d'utiliser des microsphères dont le diamètre soit compris entre 5 et 100 µm, selon les substances actives,

La substance active peut notamment être choisie parmi les stéroïdes.

Une condition essentielle pour réaliser la forme galénique selon la présente invention est de disposer de lots de microsphères calibrées, c'est-à-dire homogènes en diamètre. Si nécessaire, un tri des microsphères selon leur diamètre peut être réalisé lors de la fabrication à l'aide de procédés connus : par exemple par séparateurs cycloniques, par tamisage avec succion d'air ou encore par tamisage en milieu liquide. En pratique, il suffit que plus de 70 % des microsphères aient des diamètres compris entre 70 % et 130 % d'un diamètre spécifié. Si besoin, la courbe de dissolution idéale, déterminée par l'application envisagée, peut être approchée en effectuant un mélange de lots ayant des diamètres différents appropriés.

Des procédés pour mettre un produit solide sous forme de microsphères, par abrasion mécanique, sont connus dans l'état de la technique. D'autres procédés utilisent, par exemple, la mise en suspension du produit à l'état fondu sous forme de microgouttes, sous agitation, dans un vecteur liquide avec lequel ledit produit est non miscible, suivi de la solidification dudit produit. Le brevet WO 90/13285 décrit un procédé de fabrication de microsphères poreuses obtenues par pulvérisation, congélation et lyophilisation dans un gaz froid, de substances préalablement dissoutes dans un solvant adéquat. Pour réaliser les microsphères solides et non poreuses selon la présente invention, on a préféré développer, pour les substances qui peuvent être maintenues à l'état chimiquement stable au-dessus du point de fusion, un procédé qui consiste à pulvériser sous pression et/ou à l'aide de gaz chaud la substance (éventuellement avec des additifs) à l'état fondu, puis à congeler rapidement le brouillard ainsi formé dans un gaz froid.

De plus, les particules non conformes aux spécifications peuvent être recyclées.

Compte tenu des conditions d'utilisation, sur le plan pharmacologique, les formulations selon la présente invention sont particulièrement adaptées à des substances dont la température de fusion est supérieure à 60°C et qui sont thermostables au-dessus de leur point de fusion (ou qui peuvent être rendus thermostables à l'aide d'aditifs) pour pouvoir subir le procédé de fabrication. Un additif peut également être utilisé pour supprimer une transition de phase, d'une phase solide à une autre phase solide, succeptible de fragiliser la structure de la sphère. Le procédé est également adapté à des mélanges de substances actives en solution solide l'une dans l'autre.

La présente invention sera mieux comprise à l'aide des figures et des exemples ci-dessous. Elle n'est cependant pas limitée à ces formes d'exécution, mais seulement par la teneur des revendications.

### Brève description des figures

La figure 1 montre le schéma de fabrication des microsphères selon la présente invention.

La figure 2 montre des microsphères de progestérone (∅ moyen = 50 µm - 100 µm).

La figure 3 montre des microsphères de 17-β-estradiol (∅ moyen = 100 µm).

La figure 4 montre la répartition granulométrique d'une fraction (∅ moyen = 25 µm) de sphères de cholestérol.

La figure 5 représente un montage expérimental pour déterminer la vitesse de dissolution de microsphères.

La figure 6 montre les profils de dissolution comparés de microsphères et de cristaux de progestérone (50-125 µm).

La figure 7 montre les vitesses de dissolution comparées de microsphères et de cristaux de progestérone sous forme de dérivés de l'absorbance optique en fonction du temps.

Les figures 8 et 9 montrent les profils de dissolution comparés de microsphères et de cristaux de 17-β-estradiol ( 50 à 100 µm).

Les figures 10 et 11 montrent les profils de dissolution comparés de microsphères et de cristaux de progestérone (50 à 100 µm).

Les figures 12 et 13 montrent les profils de dissolution comparés de microsphères et de cristaux de naproxène.

Les figures 14, 15, 16 montrent les niveaux plasmatiques obtenus (chez le lapin) avec de la progestérone par injection, respectivement d'une solution huileuse, de cristaux de taille moyenne 44 µm, et de microsphères de taille moyenne 44 µm.

Les figures 17, 18, 19 montrent les niveaux plasmatiques obtenus (chez le lapin) avec du 17-β-estradiol par injection, respectivement d'une solution huileuse, de cristaux et de microsphères.

La figure 20 montre les niveaux plasmatiques obtenus (chez le lapin) avec du naproxène, respectivement d'une solution (courbe 0), de cristaux (courbe 1) et de microsphères (courbe 2).

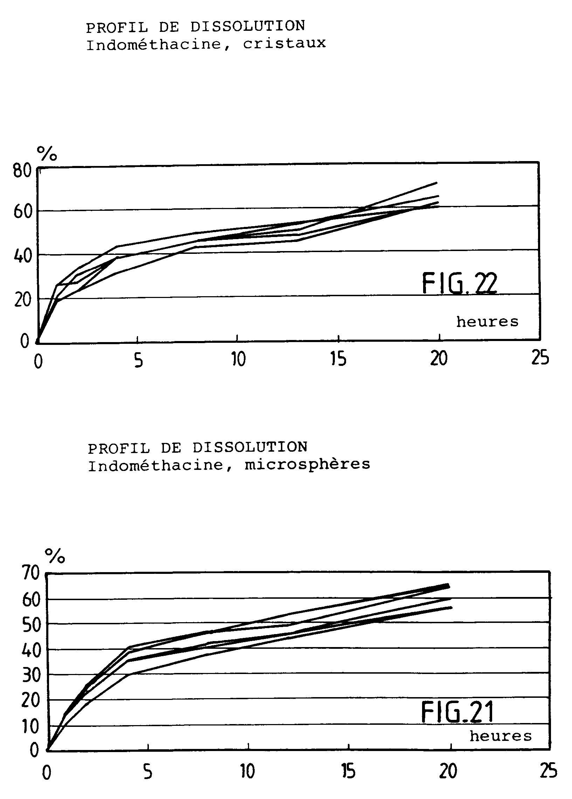

Les figures 21 et 22 montrent les profils de dissolution comparés de microsphères et de cristaux d'indométhacine (50 à 100 µm).

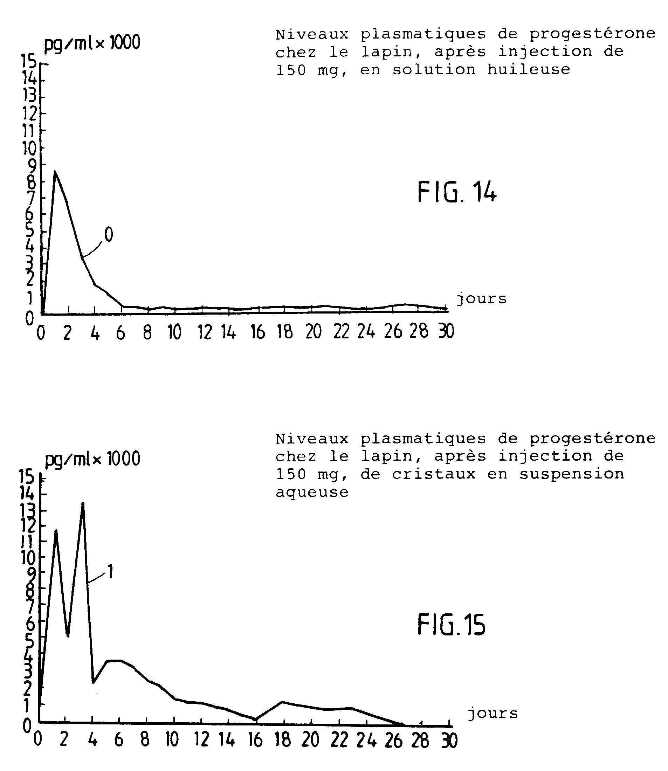

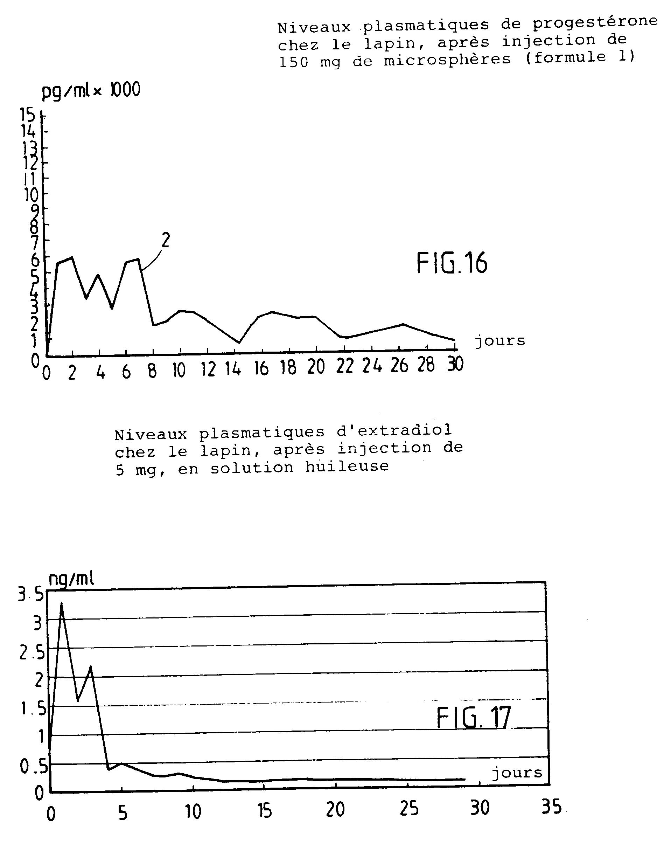

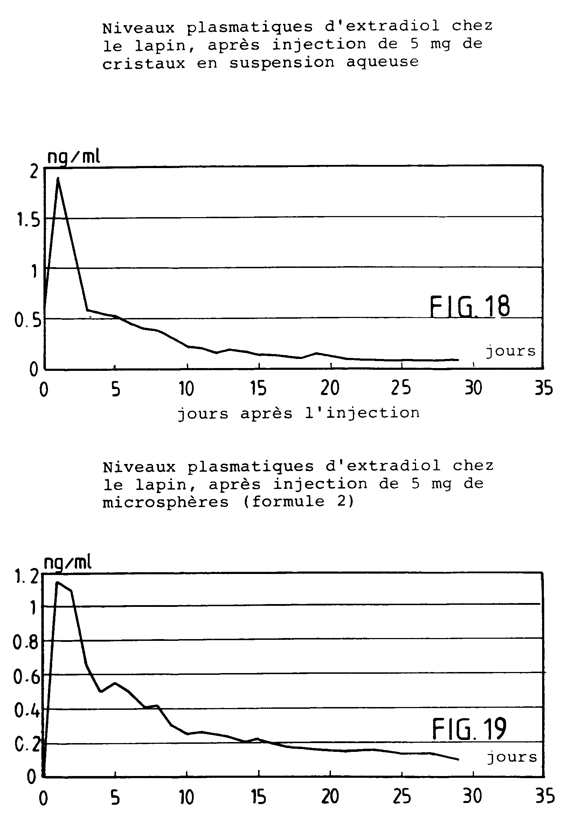

Dans les figures de 6 à 13 et 20 à 22 les abcisses sont indiquées en heures, dans les figures 14 à 19 les abcisses sont indiquées en jours, après l'injection.

### Exemple 1 : fabrication de microsphères de progestérone.

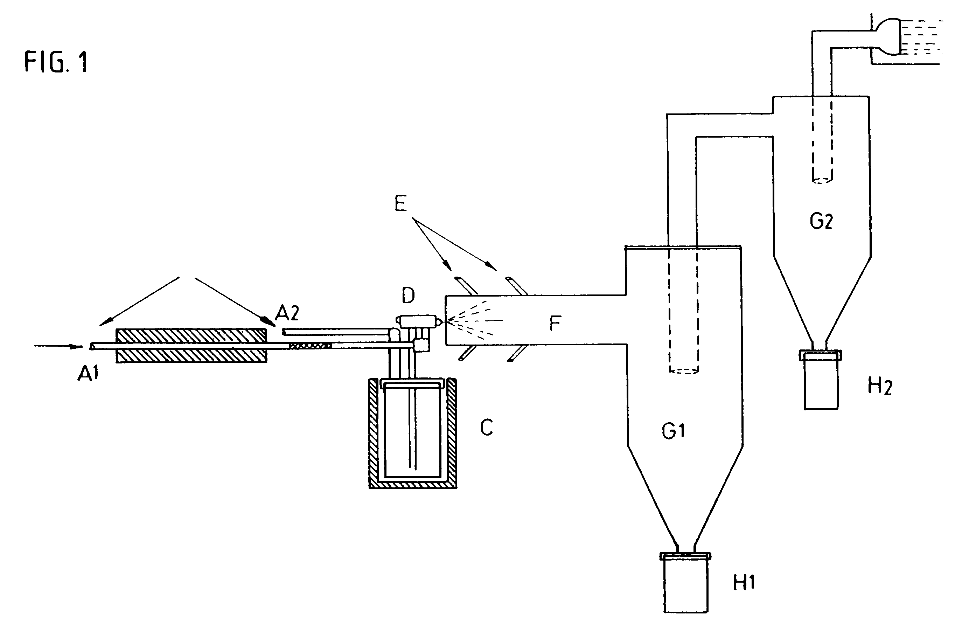

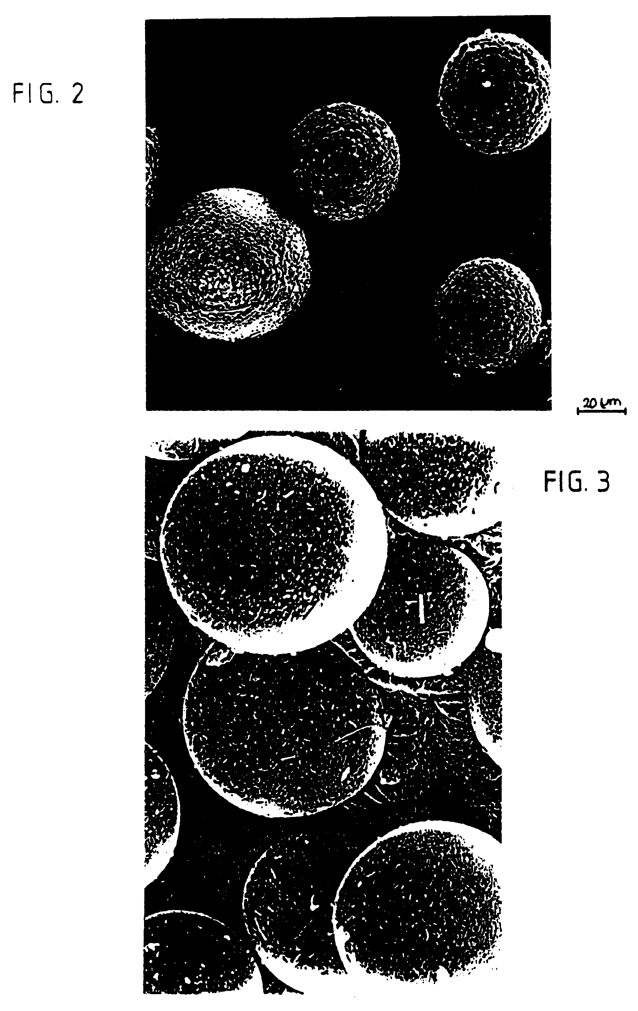

Nous nous référons à la figure 1. De l'azote préchauffé sous pression est envoyé par le tube d'entrée A₁ dans le dispositif pulvérisateur et traverse une zone de chauffage B thermo-régulée où il est porté à une température comprise entre 125 et 130°C, avant d'être admis dans le pulvérisateur D. Le pulvérisateur D est relié par une tubulure à une enceinte C chauffée dans laquelle la progestérone est maintenue à l'état fondu (T = 130°C) et sous pression d'azote (entrée A₂) . Elle est entraînée par le courant d'azote et mélangée à celui-ci, pour être pulvérisée en brouillard par la buse de sortie du pulvérisateur D et pénétre dans la chambre de pulvérisation-congélation F. Un réservoir E contient de l'azote liquide qui s'évapore et pénètre par plusieurs canalisations sous forme de gaz ultra froid, à grande vitesse, dans la chambre de pulvérisation-congélation F, où il rencontre le brouillard de progestérone. Les gouttelettes, sitôt après leur formation par le pulvérisateur sont entourées d'un courant de gaz glacial qui les cristalise en microsphères et les empêche de toucher les parois avant leur solidification totale. La température à la sortie de la chambre de pulvérisation-congélation est comprise entre - 15°C et - 50°C. La totalité des microsphères produite à l'aide de cette chambre F ont une forme sphérique parfaite. A la sortie de la chambre F se trouvent deux séparateurs cycloniques, G₁ , G₂, (de construction connue par ailleurs) montés en série. Pour un fractionnement plus fin, le nombre de cyclones peut être augmenté. Les microsphères sont récupérées dans des récipients collecteurs H₁ et H₂; les gaz, à la sortie des cyclones, traversent un filtre décontaminant I, dans lequel une légère dépression par rapport à la pression régnant dans le premier cyclone est maintenue à l'aide d'une pompe. La figure 2 montre une microphotographie d'une fraction (∅ = 50 µm à 100 µm) de microsphères de progestérone récupérées (au microscope électronique).

### Exemple 2 :

les mêmes conditions opératoires (sauf TF = 185°C) sont appliquées à la fabrication de microsphères de 17-β-estradiol avec les mêmes résultats.

La figure 3 montre une microphotographie d'une fraction de ces microsphères, de diamètre moyen 100 µm.

### Exemple 3 : Répartition granulométrique.

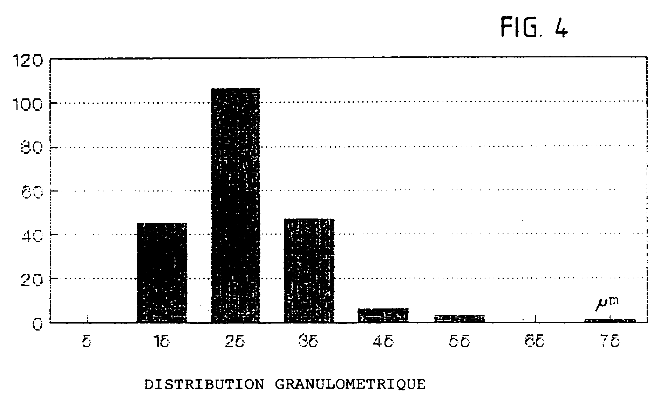

Des microsphères de cholestérol sont fabriquées par le même procédé opératoire que dans l'exemple 1. Après séparation, la fraction de diamètre moyen 25 µm présente la distribution granulométrique montrée dans la figure 4.

### Exemple 4 : Fabrication de microsphères de naproxène.

On utilise le procédé de l'exemple 1. Conditions opératoires :
Fusion : 160°C en atmosphère d'azote.
Aspersion : par valve, avec pression d'air de 0,137x10⁵ Pa (140g/cm2)
Congélation : par air à - 20°C, sous pression de 3,9 x 10⁵ Pa (4 kg/cm2)
Recouvrement : par cyclones
Sélection : en milieu acqueux et par criblage selon la granulométrie.

### Exemple 5 : microsphères de progestérone

On utilise le procédé de l'exemple 1. Conditions opératoires :
Fusion : 130°C en atmosphère d'azote.
Aspersion : par valve, avec pression d'air de 6900 Pa (70g/m2)
Congélation : par air à - 20°C, sous pression de 3,9 x 10⁵ Pa (4 kg/cm2)
Recouvrement : par cyclones
Sélection : en milieu acqueux et par criblage selon la granulométrie.

### Exemple 6 : 17-β-estradiol

On utilise le procédé de l'exemple 1. Conditions opératoires
Fusion : 185°C en atmosphère d'azote.
Aspersion : par valve, avec pression d'air de 0,137 x 10⁵ Pa (140g/cm2)
Congélation : par air à - 10°C, sous pression de 2,9 x 10⁵ Pa (3 kg/cm2)
Recouvrement : par cyclones
Sélection : en milieu acqueux et par criblage selon la granulométrie.

### Exemple 7 : microsphères d'indométhacine

On utilise le procédé de l'exemple 1. Conditions opératoires :
Fusion : 165°C en atmosphère d'azote.
Aspersion : par valve, avec pression d'air de 0,108 x 10⁵ Pa (110g/cm2)
Congélation : par air à - 20°C, sous pression de 3,9 x 10⁵ Pa (4 kg/cm2)
Recouvrement : par cyclones
Sélection : en milieu acqueux et par criblage selon la granulométrie.

Analyses comparatives spectrophotométrique UV et IR avant et après formation de microsphères.

Il est nécessaire de vérifier qu'aucune dégradation chimique des substances n'intervient au cours du processus de fusion-congélation, qui puisse modifier leur action thérapeutique. La comparaison se fait entre la matière première (cristaux) et les microsphères obtenues par fusion-congélation, par analyse spectrophotométrique en UV et en IR. Les graphiques "avant et après" doivent toujours être superposables en UV et généralement IR. Lorsqu'il apparait des différences dans les courbes d'infrarouge, il convient de vérifier si elles n'ont pas été causées par un phénomène de polymorphisme, en ayant recours à la chromatographie liquide de haute résolution avec arrangement de diodes. Il convient également de recourir à la thermographie, non seulement pour bien cerner les points de fusion, mais aussi pour déterminer s'il y apparaissent des endothermies ou exothermies qui peuvent aussi bien exprimer des modifications structurelles ou un polymorphisme, susceptibles d'avoir un effet sur le processus de formation des microsphères, que les dégradations chimiques produites par le chauffage.

Appareil utilisé en spectrographie en ultraviolet : Hewlett Packard modèle 8452A avec arrangement de photodiodes, avec cellule de quartz ayant un faisceau de 0,1 cm.

Solvents : ethanol pour le 17-β-estradiol, la progestérone et le cholestérol; HC1 à 0,1 N pour le naproxene, NaOH à 0,1 pour l'indométhacine.

Les résultats ne montrent pas de trace d'alération.

Appareil utilisé en spectrophotométrie en infrarouge : Beckmann Acculab 10. Milieu de dispersion : bromure de potasse

Chromatographe : liquide de haute résolution avec détection par arrangement de photodiodes : Waters 990 et N.E.C. Powermate 2 workstation.

Les résultats ne montrent aucune altération après la mise en microsphères pour l'indométhacine, la progestérone, le 17-β-estradiol et le naproxene.

Thermographe : SHIMADZU DSC-50 Calormeter et CR4A work-station.

Les points de fusion relevés sur les thermogrammes différentiels, montrent qu'il n'y a pas eu d'altération chimique des substances (exemple : TF cristaux : 130°C, TF microsphères : 129°C pour la progestérone). Les thermogrammes de la progestérone et du 17-β-estradiol montrent seulement une modification morphologique des phases cristallines solides.

### Exemple 8 : Courbes de dissolution de microsphères de progestérone.

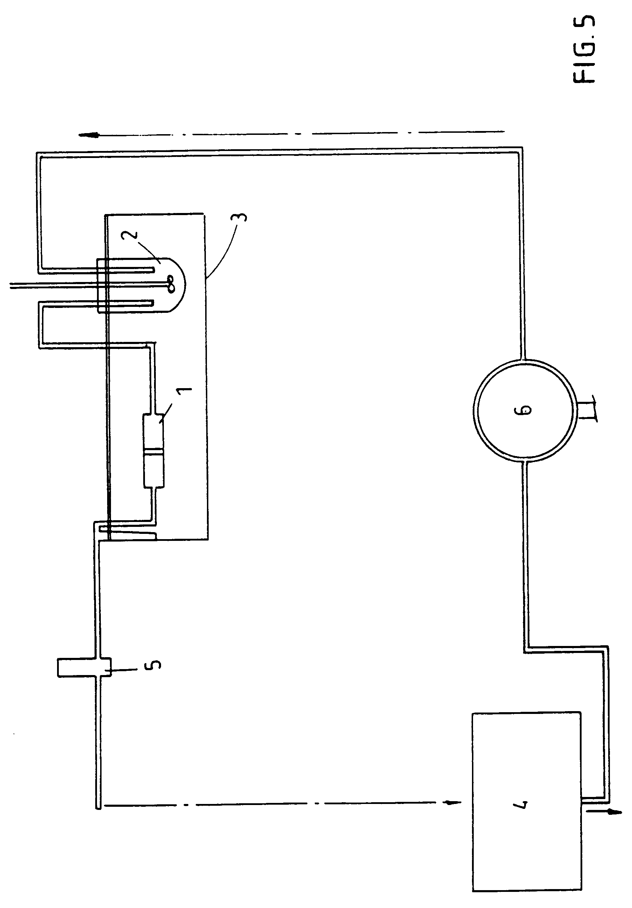

Les essais peuvent menés soit dans l'eau pure, soit dans un milieu eau/polypropylène-glycol 1:1 pour accélérer la dissolution. Le montage expérimental est montré par la figure 5. Une cellule à perfusion 1, contenant l'échantillon, est alimentée par un réservoir (agité) de milieu de dissolution 2; les deux sont contenus dans un bain-marie 3. La densité optique du milieu, à 240 nm, est enregistrée par un spectrophotomètre 4 et le milieu est ramené dans le réservoir. Un piège à bulle 5 et une pompe péristaltique 6 complètent le circuit.

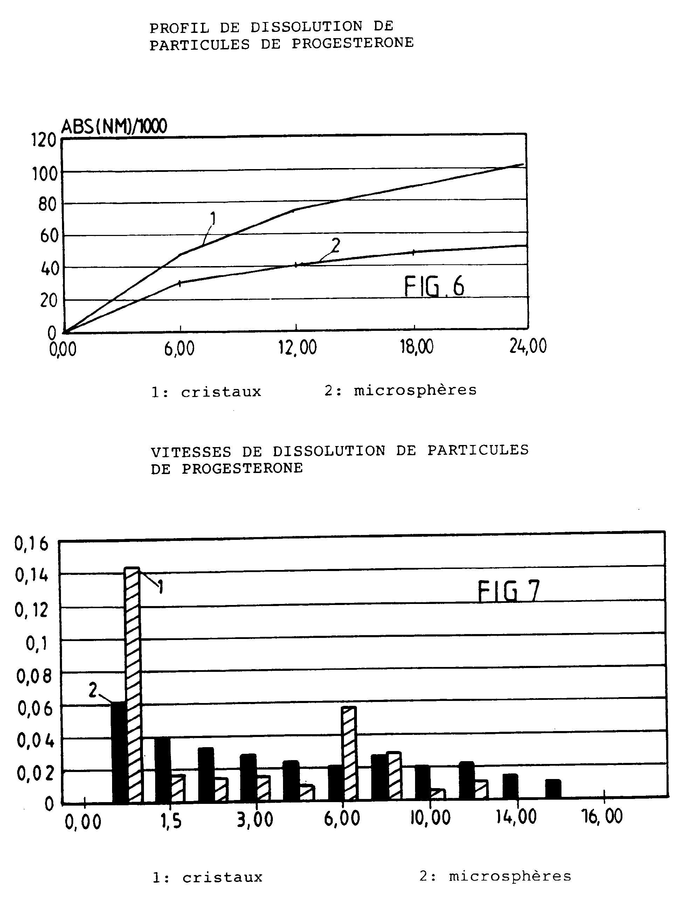

La figure 6 montre les profils de dissolution comparés de microsphères (courbe 2) et de cristaux (courbe 1) de granulométries comprises entre 50 et 125 µm, mesurés par la variation d'absorbance optique en fonction du temps. L'essai est effectué dans un milieu eau/PPG 50:50. On constate que la dissolution est retardée par la mise en forme de microsphères.

La figure 7 montre les vitesses de dissolution (dérivées des variations de D.O. en fonction du temps) de cristaux (1) et de microsphères (2) de même granulométrie moyenne (environ 150 µm). La distribution granulométrique des cristaux est plus hétérogène et leur courbe de dissolution plus irréguliere que celle des microsphères.

Les exemples suivants montrent la reproductibilité comparée des parties initiales des courbes de dissolution de cristaux et de microsphères de granulométrie comparable, d'un même produit. L'appareil utilisé est celui de la figure 5. Plusieurs (de 3 - 6) circuits de mesure (cellules de dissolution et tubulures) contenant des échantillons identiques, sont mis en oeuvre en parallèles par la même pompe péristaltique et mesurés simultanément.

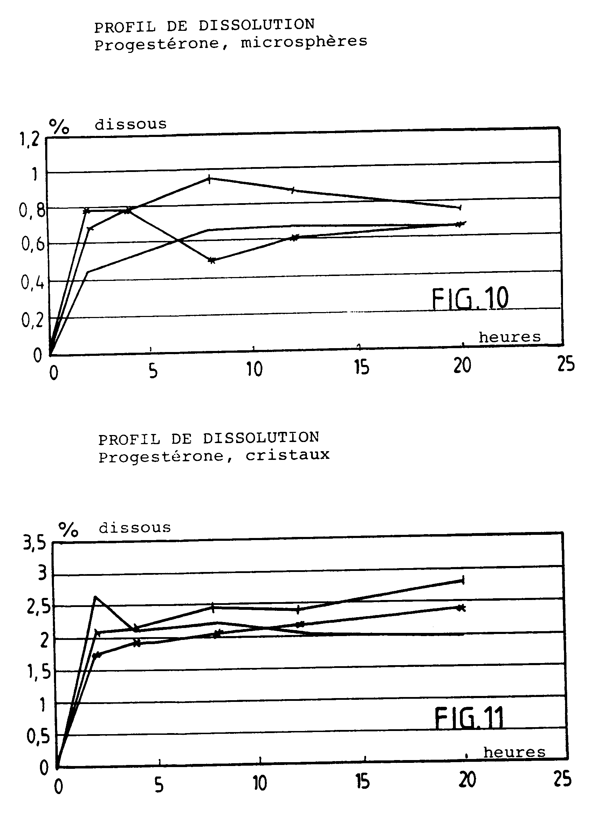

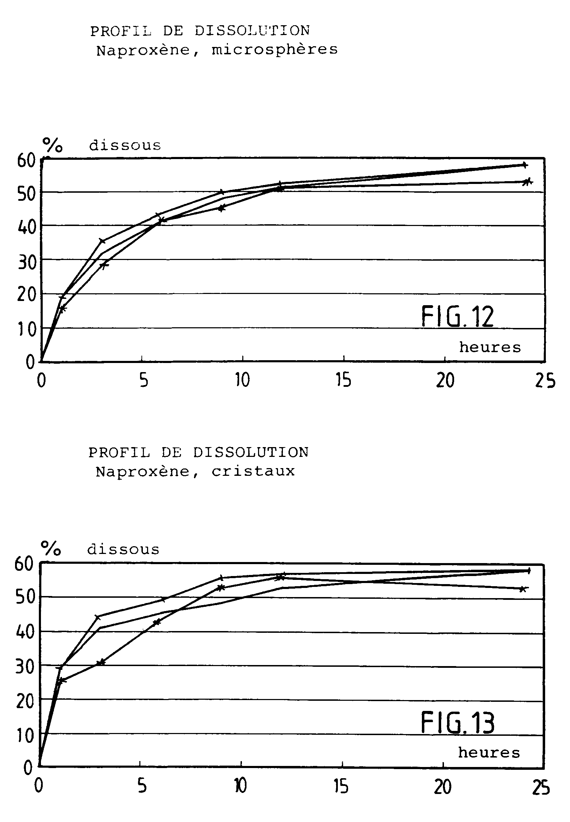

### Exemple 10 : Dissolution de cristaux de progestérone (fig. 11) / microsphères de progestérone (fig. 10).

Milieu de dissolution utilisé : H₂O qualité HPLC avec 0,01 **%** de Tween 80
Echantillon : 50 mg.
granulométrie : 50 à 100 µm
Intervalles d'échantillonage : 0, 2, 4, 8, 14,20 heures
Longueur d'ondes de spectrophotométrie : 240 nm

### Exemple 11. Dissolution de microsphères de naproxene (fig. 12) / cristaux de naproxene (fig. 13). L'appareil utilisé est celui de la figure N° 5.

Milieu de dissolution utilisé : H₂O qualité HPLC avec 0,01 **%** de Tween 80
Echantillon : 50 mg.
granulométrie : 50 à 100 µm
Intervalles d'échantillonage : 0,1,3,6,9,12,24 heures
Longueur d'ondes de spectrophotométrie : 232 nm

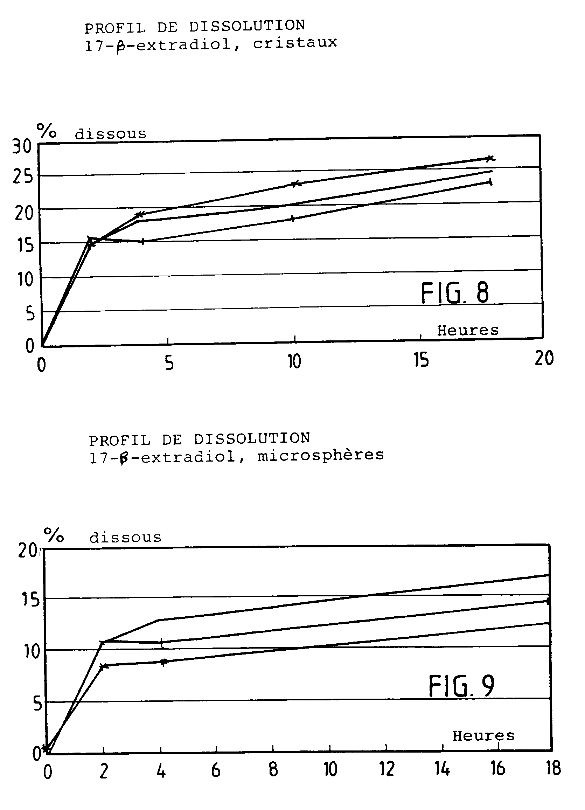

### Exemple 12 : Dissolution de microsphères de 17-β-estradiol : (Fig. 9) / cristaux de 17-β-estradiol (fig. 8). L'appareil utilisé est celui de la figure N° 5.

Milieu de dissolution utilisé : H₂O qualité HPLC avec 0,01 % de Tween 80
Echantillon : 50 mg.
granulométrie : 50 à 100 µm
Intervalles d'échantillonage : 0, 2, 4, 18 heures
Longueur d'ondes de spectrophotométrie : 282 nm

L'ensemble des courbes montre que la reproductibilité des résultats et la régularité des profils de dissolution est meilleure pour des lots de microsphères que pour des lots de cristaux, dans la phase initiale de dissolution (qui est la période la plus critique).

### Exemple 14 : Formulations injectables

### Formule N° 1

- Microsphères de progestérone: 75 mg
- Polyéthylène Glycol 800: 20 mg
- Carboxyméthylcélulose sodique: 1.66 mg
- Polysorbate 80: 2.0 mg
- Propylparabène: 0.14 mg
- NaCl: 1.2 mg
- H₂O qsp: 1 ml

### Formule N° 2

- Microsphères de 17-β-estradiol: 2.5 mg
- Polyéthylène Glycol 800 20: 20 mg
- Carboxyméthylcélulose sodique: 1.66 mg
- Polysorbate 80: 2.0 mg
- Propylparabène: 0.14 mg
- NaCl: 1.2 mg
- H₂O qsp: 1 ml

### Formule N° 3.

- Microsphères de Naproxene: 100 mg
- Carboxyméthylcélulose sodique: 5.0 mg
- Polysorbate 80: 4.0 mg
- NaCl: 9.0 mg
- Alcool de benzyl: 9.0 mg
- H₂O qsp: 1 ml

### Exemple 15 : Etude des niveaux plasmatiques de progestérone chez le lapin (fig. 14, 15, 16).

L'étude comprend l'évaluation comparée de l'effet sur les niveaux plasmatiques chez le lapin, produit par l'administration parentérale de progestérone sous forme d'une solution huileuse (0), d'une suspension aqueuse de cristaux (1) et d'une suspension aqueuse de microsphères (2) (formule N° 1, granulométrie moyenne : 44 µm)

A 10 lapins mâles de race Nouvelle Zélande d'un poids moyen de 3,5 Kg on administre une dose unique intramusculaire de 150 mg de progestérone (2 ml).

L'intervalle d'échantillonage est de 1, 2, 4 et 24 heures durant 20 jours, puis à chaque trois jours jusqu'à atteindre 30 jours.

Les prises sont de 2ml par venoponction, sont centrifugées, puis gardées à -20°C jusqu'à l'analyse par radioimmunoanalyse.

### Exemple 16 : Etude des niveaux plasmatiques d'estradiol chez le lapin.

L'étude comprend l'évaluation comparée de l'effet sur les niveaux plasmatiques chez le lapin, produit par l'administration parentérale d'estradiol en forme d'une solution huileuse (0), d'une suspension aqueuse de cristaux (1) et d'une suspension aqueuse de microsphères d'estradiol (2) (formule N° 2, granulométrie 50-100 µm).

A 8 lapins mâles de race Nouvelle Zélande d'un poids moyen de 3,5 Kg on administre une dose unique intramusculaire contenant 5 mg d'estradiol (2 ml).

L'intervalle d'échantillonage est de 1, 2, 4 et 24 heures durant 20 jours, puis à chaque trois jours jusqu'à atteindre 30 jours.

Les prises sont de 2ml par venoponction, sont centrifugées, puis gardées à -20°C jusqu'à l'analyse par radioiminunoanalyse.

### Exemple 17 : Evolution comparative des niveaux plasmatiques de naproxene en solution huileuse et en suspension de microsphères.

Sujets d'expérimentation : lapins de race Nouvelle-Zélande agés d'environ 5 mois et pesant en moyenne 3,7 kg.

La prise de référence est de 5 ml de sang par ponction cardiaque, suivie de l'administration intramusculaire de 2ml de la formule à tester (formule 3) dans le membre inférieur droit.

Les prises à analyser furent prélevées à intervalles de 30 minutes durant 2 heures et à intervalles de 60 minutes jusqu'à compléter 6 heures. Dans plusieurs essais, en fonction des caractéristiques cinétiques du médicament, il y eut des prises additionnelles.

Des prises à analyser de 2ml, également prélevées par ponction cardiaque, furent placées en Vacutainer, additionnées d'héparine, centrifugées à 3000 rpm durant 10 min., puis le plasma séparé et congelé en cryotubes à -20°C jusqu'à son analyse.

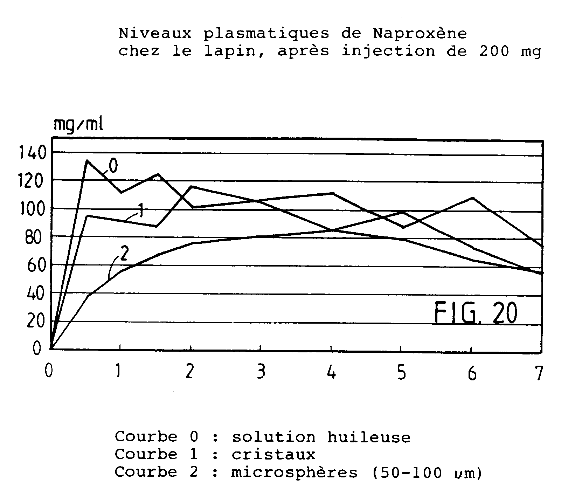

La figure 20 montre que la variation des niveaux plasmatique, atteints après injection de microsphères, est beaucoup plus régulière que celle obtenue après injection de particules de forme quelconque (50-100 µm).

En résumé, l'ensemble des résultats ci-dessus montre que dans la phase initiale de dissolution, des substances pharmaceutiquement actives présentent des valeurs numériques plus reproductibles et un profil de dissolution plus régulier lorsqu'elles sont sous forme d'échantillon de micro-sphères calibrées que sous forme de particules de formes irrégulières. Ceci permet de calculer de manière plus précise une dose pharmaceutiquement efficace. De plus, la disparition, ou du moins la forte diminution du pic initial de dissolution (par rapport à des cristaux ou des particules quelconques) ainsi que le ralentissement et la prolongation globale du phénomène de dissolution permet de calculer des doses unitaires plus importantes destinées à être administrées à des intervalles de temps plus espacés.

D'autre part, les résultats ci-dessus montrent que l'utilisation de ce type de structure convient aussi bien à la fabrication des médicaments dont la durée d'action est relativement courte, quelques heures à quelques jours (p.e. analgésiques) qu'à des substances dont la durée d'action envisagée est de plusieurs semaines. Parmi ces dernières on peut citer en particulier l'utilisation d'hormones sexuelles (comme la progestérone et le 17-β-estradiol) pour la fabrication d'anticonceptifs destinés à une injection mensuelle ou d'anticonceptifs destinés plus particulièrement à la femme post-partum, ou encore pour la fabrication de médicaments à longue durée d'action, injectables, destinés à la prévention de l'ostéoporose chez la femme ménopausée.

Le procédé de fabrication décrit ci-dessus, les structures sphériques et les formulations obtenues et leur utilisation par voie parentérale par injection ne sont bien entendu pas limitées aux substances données en exemples ci-dessus, mais sont applicables à toutes substances pharmacologiquement actives, chimiquement stables pendant la micronisation, à condition que les modifications pharmacocinétiques que permettent les microsphères (durée brève ou longue selon le diamètre, régularisation des profils plasmatique), présentent un avantage thérapeutique ou de commodité et que les doses à administrer ne dépassent pas un volume raisonnable. On peut choisir le mode d'administration parmi l'injection hypodermique, l'injection sous cutanée, l'injection intramusculaire, l'injection intra-articulaire et l'injection intra-rachidienne, selon l'application envisagée.

## Revendications

1. Microsphère solide, non poreuse, d'un diamètre compris entre 1 µm et 300 µm constituée d'au moins une substance pharmaceutiquement active injectable et sans excipient à point de fusion inférieur à 60°C.

2. Microsphère selon la revendication 1, caractérisée en ce que le diamètre de ladite microsphère est compris entre 5 et 100 µm.

3. Microsphère selon la revendication 2, caractérisée en ce que son point de fusion est supérieur à 60°C.

4. Microsphère selon la revendication 3, caractérisée en que sa composition comprend également des additifs pharmaceutiquement acceptables.

5. Microsphère selon la revendication 1, susceptible d'être obtenue par pulvérisation de ladite substance à l'état fondu et congélation rapide des gouttelettes dans un gaz froid.

6. Microsphère selon la revendication 1, caractérisée en ce que la substance pharmaceutique active est choisie parmi les stéroïdes.

7. Microsphère selon la revendication 5, caractérisée en ce que ladite substance pharmaceutiquement active est la progestérone.

8. Microsphère selon la revendication 5, caractérisée en ce que ladite substance pharmaceutiquement active est le 17-β-estradiol.

9. Microsphère selon la revendication 1, caractérisée en ce que ladite substance pharmaceutiquement active est choisie parmi les analgésiques.

10. Microsphère selon la revendication 5, caractérisée en ce que ladite substance pharmaceutiquement active est le naproxene.

11. Microsphère selon la revendication 5, caractérisée en ce que ladite substance pharmaceutiquement active est l'indomethacine.

12. Procédé pour améliorer le contrôle des propriétés pharmacocinétiques et pharmacologiques d'une substance pharmaceutiquement active injectable, consistant à mettre ladite substance sous forme de microsphères selon l'une quelconque des revendications 1 à 11 et à séparer lesdites microsphères en fractions calibrées selon leurs diamètres.

13. Procédé selon la revendication 12, caractérisé en ce que la séparation en fractions est effectuée de façon à ce que plus de 70 % desdites microsphères aient des diamètres compris entre 70 % et 130 % d'un diamètre spécifié.

14. Procédé selon la revendication 13 comprenant les étapes suivantes :
a. Fusion de la substance active sous atmosphère inerte.
b. Pulvérisation en brouillard de gouttelettes sous pression d'atmosphère inerte.
c. Congélation en atmosphère froide.
d. Tri par fractions granulométriques.

15. Utilisation de microsphères selon l'une quelconque des revendications 1 à 11, calibrées, pour la fabrication d'une formulation destinée à l'administration parentérale par injection.

16. Utilisation selon la revendication 15, caractérisée en ce que le mode d'administration est choisi parmi l'injection hypodermique, l'injection sous-cutanée, l'injection intramusculaire, l'injection intra-articulaire et l'injection intra-rachidienne.

17. Utilisation selon la revendication 16, caractérisée en ce que lesdites microsphères sont présentées sous forme d'une poudre, prête à être mise en suspension au moment de l'emploi dans un vecteur liquide pharmaceutiquement acceptable choisi parmi les solutions acqueuses, notamment une solution saline, et les huiles.

18. Utilisation selon la revendication 16, caractérisée en ce que lesdites microsphères sont présentées sous forme d'une suspension dans un vecteur liquide pharmaceutiquement acceptable dans lequel lesdites microsphères sont insolubles.

19. Utilisation de microsphères selon la revendication 1 pour la fabrication d'un anticonceptif destiné à l'injection parentérale, caractérisée en ce que ladite formulation comprend une association de microsphères calibrées de progestérone et de microsphères calibrées de 17-β-estradiol.

20. Utilisation de microsphères de progestérone selon la revendication 5, pour la fabrication d'un anticonceptif post partum destiné à l'injection parentérale.

21. Utilisation de microsphères de progestérone selon la revendication 5 pour la fabrication d'un médicament administrable par injection parentérale destiné à la prévention de l'ostéoporose chez la femme ménopausée.

22. utilisation de microsphères selon l'une des revendications 9, 10, 11, pour la fabrication d'un analgesique à action prolongée destiné à l'injection parentérale.

## Patentansprüche

1. Feste, nicht poröse Mikrokugel, mit einem Durchmesser zwischen 1 µm und 300 µm, die aus wenigstens einer injizierbaren pharmazeutisch aktiven Substanz, ohne Grundmasse mit einem Schmelzpunkt unter 60°C, besteht.

2. Mikrokugel gemäss Anspruch 1, dadurch gekennzeichnet, dass der Durchmesser der genannten Mikrokugel zwischen 5 und 100 µm beträgt.

3. Mikrokugel gemäss Anspruch 2, dadurch gekennzeichnet, dass ihr Schmelzpunkt höher als 60°C liegt.

4. Mikrokugel gemäss Anspruch 3, dadurch gekennzeichnet, dass ihre Zusammensetzung ebenfalls pharmazeutisch annehmbare Zusatzsubstanzen einbegreift.

5. Mikrokugel gemäss Anspruch 1, herstellbar durch Zerstäubung der genannten Substanz in geschmolzenem Zustand und durch schnelles Einfrieren der Tröpfchen in einem kalten Gas.

6. Mikrokugel gemäss Anspruch 1, dadurch gekennzeichnet, dass die pharmazeutisch aktive Substanz aus den Steroiden ausgewählt ist.

7. Mikrokugel gemäss Anspruch 5, dadurch gekennzeichnet, dass die genannte pharmazeutisch aktive Substanz Progesteron ist.

8. Mikrokugel gemäss Anspruch 5, dadurch gekennzeichnet, dass die genannte pharmazeutisch aktive Substanz 17-β-Estradiol ist.

9. Mikrokugel gemäss Anspruch 1, dadurch gekennzeichnet, dass die genannte pharmazeutisch aktive Substanz zwischen den schmerzstillenden Substanzen ausgesucht ist.

10. Mikrokugel gemäss Anspruch 5, dadurch gekennzeichnet, dass die genannte pharmazeutisch aktive Substanz Naproxen ist.

11. Mikrokugel gemäss Anspruch 5, dadurch gekennzeichnet, dass die genannte pharmazeutisch aktive Substanz Indomethacin ist.

12. Verfahren um die Kontrolle über die pharmakinetischen und pharmakologischen Eigenschaften einer injiziebaren pharmazeutisch aktiven Substanz zu verbessern, in dem die genannte Substanz in Form von Mikrokugeln gemäss irgend einem der Ansprüche 1 bis 11 gebracht wird und die genannten Mikrokugeln ihrem Durch- messer nach in kalibrierte Fraktionen aufgetrennt werden.

13. Verfahren gemäss Anspruch 12, dadurch gekennzeichnet, dass die Auftrennung in Fraktionen so erfolgt, dass mehr als 70% der genannten Mikrokugeln Durchmesser aufweisen, die zwischen 70% und 130% eines vorgeschriebenen Durchmessers betragen.

14. Verfahren gemäss Anspruch 13, mit folgenden Schritten:
a) Schmelzen der aktiven Substanz unter inerter Atmosphäre
b) Zerstäuben in einen Tröpfchennebel, unter Druck, in inerter Atmosphäre
c) Einfrieren in kalter Atmosphäre
d) Auftrennung in korngrössenmässigen Fraktionen

15. Verwendung von kalibrierten Mikrokugeln gemäss irgend einem der Ansprüche 1 bis 11, zur Herstellung eines Arzneimittels, welches zur parenteralen Verabreichung durch Injizieren bestimmt ist.

16. Verwendung gemäss Anspruch 15, dadurch gekennzeichnet, dass die Verabreichungsart zwischen der Unterhaut-, der Subkutanen-, der Intramuskularen-, der Intraartikularen- und der Intravirbelkanalinjektion ausgewählt ist.

17. Verwendung gemäss Anspruch 16, dadurch gekennzeichnet, dass die genannten Mikrokugeln in Form eines Pulvers aufbereitet werden, das fertig zum Suspendieren, zur Gebrauchszeit, in einem pharmazeutisch annehmbaren flüssigen Träger ist, der zwischen den wässrigen Lösungen, insbesondre den Salzlösungen, und den Oelen ausgewählt wird.

18. Verwendung gemäss Anspruch 16, dadurch gekennzeichnet, dass die genannten Mikrokugeln in Form einer Suspension, in einem pharmazeutisch annehmbaren flüssigen Träger, in welchem die genannten Mikrokugeln unlösbar sind, dargeboten werden.

19. Verwendung von Mikrokugeln gemäss Anspruch 1 zur Herstellung eines empfängnisverhütenden Mittels, das zur Parenteral-Injektion bestimmt ist, dadurch gekennzeichnet, dass das genannte Mittel eine Assoziation von kalibrierten Progesteron Mikrokugeln und kalibrierten 17-β-Estradiol Mikrokugeln enthält.

20. Verwendung von Progesteron Mikrokugeln gemäss Anspruch 5, zur Herstellung eines post-partum Empfängnisverhütungsmittels, das zur parenteralen Injektion bestimmt ist.

21. Verwendung von Progesteron Mikrokugeln gemäss Anspruch 5, zur Herstellung eines, durch parenterale Injektion verabreichbaren Arzneimittels, das zur Verhütung der Osteoporose der Frau nach den Wechseljahren bestimmt ist.

22. Verwendung von Mikrokugeln gemäss einem der Ansprüche 9, 10 oder 11 zur Herstellung eines langwirkenden schmerzstillenden Arzneitmittels, das zur parenteralen Injektion bestimmt ist.

## Claims

1. Solid, non-porous microsphere of a diameter between 1 and 300 µm, consisting of at least one injectable pharmaceutically active substance, without an excipient having a melting point lower than 60°.

2. Microsphere according to Claim 1, characterised in that the diameter of the said microspheres is between 5 and 100 µm.

3. Microsphere according to Claim 2, characterised in that its melting point is greater than 60°C.

4. Microsphere according to Claim 3, characterised in that its composition also comprises pharmaceutically acceptable additives.

5. Microsphere according to Claim 1, obtainable by spraying the said substance in the melted state and rapidly freezing the droplets in a cold gas.

6. Microsphere according to Claim 1, characterised in that the said pharmaceutically active substance is selected from steroids .

7. Microsphere according to Claim 5, characterised in that the said pharmaceutically active substance is progesterone.

8. Microsphere according to Claim 5, characterised in that the said pharmaceutically active substance is 17-β-estradiol.

9. Microsphere according to claim 1, characterized in that the said pharmaceutically active substance is selected from analgesics.

10. Microsphere according to Claim 5, characterised in that the said pharmaceutically active substance is naproxen.

11. Microsphere according to Claim 5, characterised in that the said pharmaceutically active substance is indomethacin.

12. Process for improving the control of the pharmacokinetic and pharmacological properties of an injectable pharmaceutically active substance, consisting in preparing the said substance in the form of solid, non-porous microspheres according to any one of claims 1-11, and in separating the said microspheres into calibrated fractions according to their diameter.

13. Process according to Claim 11, characterised in that the separation in fractions is performed until more than 70% of the said microspheres have diameters between 70% and 130% of a specified diameter.

14. Process according to Claim 13, comprising the following stages:
a. Melting of the active substance under inert atmosphere.
b. Spraying into a cloud of droplets under pressure in an inert atmosphere.
c. Freezing in a cold inert atmosphere.
d. Separation according to size fractions.

15. Use of the microspheres according to any one of Claims 1 to 11, in calibrated form, for the manufacture of a formulation intended for parenteral administration by injection.

16. Use according to Claim 15, characterised in that the mode of administration is chosen from among hypodermic injection, subcutaneous injection, intramuscular injection, intra-articular injection and intra-rachidian injection.

17. Use according to Claim 16, characterised in that the said microspheres are provided in the form of a powder ready for preparing into a suspension at the time of use in a pharmaceutically acceptable liquid vector chosen from among aqueous solutions, in particular a saline solution, and oils.

18. Use according to Claim 16, characterised in that the said microspheres are provided in the form of a suspension in a pharmaceutically acceptable liquid vector in which the said microspheres are insoluble.

19. Use of the microspheres according to Claim 1 for the manufacture of a contraceptive intended for parenteral injection characterised in that the said formulation comprises a combination of calibrated progesterone microspheres and calibrated 17-β-estradiol microspheres.

20. Use of progesterone microspheres according to Claim 5 for the manufacture of a postpartum contraceptive intended for parenteral injection.

21. Use of progesterone microspheres according to Claim 5, for the manufacture of a medicinal product for parenteral injection intended for the prevention of osteoporosis in menopausal women.

22. Use of the microspheres according to any of claims 9, 10, 11, for the manufacture of a slow-release analgesic, intended for parenteral injection.
